# EUROPEAN PATENT APPLICATION

(11) **EP 2 636 682 A1**
(43) Date of publication of application: **11.09.2013**
(21) Application number: 12158931.1
(22) Date of filing: 09.03.2012
(51) Int. Cl.: C07K 16/06, C07K 14/435, A61K 39/395, A61P 31/04

(54) **Compositions comprising secretory-like immunoglobulins**

(71) Applicant: CSL Behring AG, 3000 Bern 22 (CH)
(72) Inventor: The designation of the inventor has not yet been filed
(74) Representative: Peter, Beate

(57) **Abstract**

The invention relates to methods for preparing compositions comprising secretory-like immunoglobulin, in particular secretory-like IgA and/or secretory-like IgM, and compositions obtainable by the methods.

## Description

The invention relates to methods for preparing compositions comprising secretory-like immunoglobulin, in particular secretory-like IgA and/or secretory-like IgM, and compositions obtainable by the methods.

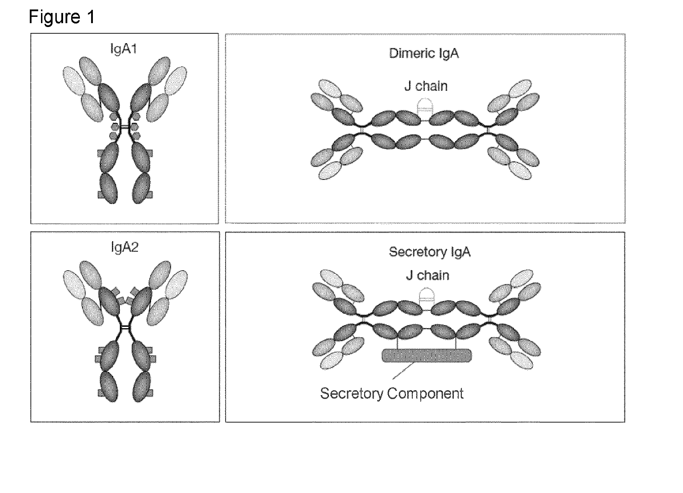

IgA is the second most abundant Ig class after IgG in human plasma where it is found at 0.88-4.10 g/L. There are two subclasses of IgA, IgA1 and IgA2 (Fig. 1) (Zuercher AW et al. Plasma derived immunoglobulins. Principles of Immunopharmacology. 3rd ed. Birkhäuser, 2011: p. 271-301) which differ in their disulfide bonds linking heavy and light chains as well as in their antigenic diversity due to significant differences in the hinge region of the molecule. The glycosylation pattern of the two subclasses is different: the heavy chain of both subclasses is N-glycosylated; in contrast, O-glycosylation is found on IgA1 but not IgA2, due to the truncated hinge region of IgA2.

Human IgA is found in two major forms: either circulating in blood/ plasma, or secreted to mucosal surfaces. In plasma, IgA is predominantly present as monomers (80-90%) and is produced by bone marrow plasma cells; the major subclass in plasma is IgA1.

The term polymeric IgA (plgA) describes dimeric, occasionally tetrameric, IgA covalently joined at their "tail-pieces" by the Joining (J) chain (Fig. 1).

IgM molecules make up 10% of the total serum Ig content. They are confined predominantly to the intravascular pool and are part of the primary, antigen-specific, humoral immune response; phylogenetically and ontogenetically they are the earliest antibody (Ab) molecules. IgM exists predominantly as a pentamer joined by the J chain and arranged into a planar structure; occasionally, IgM can also be found in a hexameric form lacking the J chain (Zuercher AW et al. Plasma derived immunoglobulins. Principles of Immunopharmacology. 3rd ed. Birkhäuser, 2011: p. 271-301).

Mucosal surfaces of the digestive, respiratory and urogenital tracts, as well as the ducts of exocrine glands are lined by layers of epithelial cells that form a tight barrier separating the body's internal compartments from the outside environment. In humans, these vast surfaces cover 400 m², an area that is permanently exposed to exogenous pathogens (Corthésy, B. (2010) Future Microbiol. 5:817-829). The combination of innate and inducible cellular and molecular mechanisms ensures protection against colonization and entry/invasion by microbes. In healthy individuals, secretory IgA (SlgA) is the most abundant antibody (Ab) fulfilling the function of immune exclusion on the luminal side of mucosal surfaces (Macpherson, A.J. et al. (2008) Mucosal Immunol. 1:11-22), whereas secretory IgM Abs take over in IgA-deficient patients. Secretory (S)IgA is synthesised by mucosal plasma cells of the intestinal lamina propria, the upper respiratory tract or the uro-genital tract. SlgA consists of a pIgA dimer, and a secretory component (SC) of approximately 70 kDa (Fig. 1); similarly pentameric, J chain-containing IgM associated with SC constitutes SlgM. The SC represents the extracellular part of the polymeric Ig receptor (pIgR). pIgR is needed for the trans-epithelial transport of pIgA or pentameric IgM from the site of production to the mucosal surface where the pIgR-plgA / plgR-IgM complex is converted to SlgA / SlgM by enzymatic cleavage (Zuercher AW et al. Plasma derived immunoglobulins. Principles of Immunopharmacology. 3rd ed. Birkhäuser, 2011: p. 1-31). Association with SC protects IgA or IgM from proteolytic degradation. SlgA is the predominant Ig in seromucous secretions such as saliva, trachea-bronchial secretions, colostrum, milk, tear fluid, intestinal secretions and uro-genital secretions. It is the most prominent Ig produced at mucosal linings (and thus in the human body); approx. 3-5 g of SlgA is secreted daily into the intestinal lumen. SlgA is thus essential for immune exclusion and to maintain epithelial integrity. SlgM is present at lower levels but fulfills the same immune exclusion functions as SlgA.

For a few pathogens such as Poliovirus, *Salmonella,* or influenza, protection against mucosal infection can be induced by active mucosal immunization with licensed vaccines. However, for the majority of mucosal pathogens no active mucosal vaccines are available. Alternatively, protective levels of Abs might directly be delivered to mucosal surfaces by passive immunization. In nature this occurs physiologically in many mammalian species by transfer of maternal antibodies to their offspring via milk (Brandtzaeg, P. (2003) Vaccine 21:3382-3388). Human and animal studies using passive mucosal immunization have demonstrated that plgA and SlgA antibody molecules administered by oral, intranasal, intrauterine or lung instillation can prevent, diminish, or cure bacterial and viral infections (Corthésy, B. (2003) Curr. Pharm. Biotechnol. 4:51-67). However, the secretory form of IgA naturally found at mucosal surfaces was rarely used, and large scale production of SlgA is not possible to date. Construction of SlgA with biotechnological methods is challenging but such molecules could have important clinical applications (Corthésy, B. (2002) Trends Biotechnol. 20:65-71). The same also applies to secretory component-containing IgM.

### Summary of the invention

The inventors have surprisingly found that it is possible to combine plasma-derived J chain-containing immunoglobulin, in particular IgA and/or IgM, with secretory component without the need to first purify the J chain-containing immunoglobulin. This invention opens the door to large-scale production of secretory-like IgA and/or IgM which can be used in medicine, for example for the prevention and treatment of infections on mucosal surfaces in subjects, in particular in human subjects.

One aspect of the invention is a method for producing a composition comprising secretory-like immunoglobulin, in particular IgA and/or IgM, in vitro, comprising the steps of
(a) Obtaining a blood-derived protein composition comprising J chain-containing immunoglobulin, in particular IgA and/or IgM, in a non-purified form; and
(b) admixing the composition of step (a) with secretory component.

Preferably, the secretory-like immunoglobulin is secretory-like IgA. Preferably, the composition of step (a) contains at least 5% J chain-containing IgA, more preferably at least 10%, more preferably at least 20%, even more preferably at least 30%, most preferably it will contain at least 50% J chain-containing IgA. Preferably, the composition of step (a) is derived from human blood, e.g. human plasma or fractions thereof enriched for IgA or even J chain-containing IgA, but no purification of J chain-containing IgA is required.

Preferably, the secretory-like immunoglobulin is secretory-like IgM. Preferably, the composition of step (a) contains at least 5% J chain-containing IgM, more preferably at least 10%, more preferably at least 20%, even more preferably at least 30%, most preferably it will contain at least 50% J chain-containing IgM. Preferably, the composition of step (a) is derived from human blood, e.g. human plasma or fractions thereof enriched for IgM or even J chain-containing IgM, but no purification of J chain-containing IgM is required.

The secretory component used in step (b) is preferably recombinant secretory component, more preferably human recombinant secretory component, preferably produced by a mammalian cell line. However, secretory component from a natural source can also be used, such as secretory component purified from milk, saliva, mucus or similar sources.

The molar ratio between secretory component and J chain within IgA dimers/polymers or IgM pentamers in the composition of step (a) ranges between 1:10 and 10:1, preferably between 1:5 and 5:1, more preferably between 1:2 and 2:1.

In another aspect of the invention, the molar ratio between secretory component and J chain within the composition of step a) ranges between 1:10 and 10:1, preferably between 1:5 and 5:1, more preferably between 1:2 and 2:1.

Another aspect of the invention is a composition comprising secretory-like IgA and/or secretory-like IgM or combinations thereof obtainable by a method of the invention. The composition may further comprise a pharmaceutically acceptable carrier or excipient.

A further aspect of the invention is the composition described above for medical use.

### Detailed description of the invention

As already mentioned above, the inventors have surprisingly found that it is possible to combine plasma-derived J chain-containing immunoglobulins, in particular J chain-containing IgA and/or J chain-containing IgM with secretory component without the need to first purify the J chain-containing immunoglobulin. This invention opens the door to large-scale production of secretory-like IgA and/or secretory-like IgM which can be used in medicine, for example for the prevention and treatment of infections on mucosal surfaces in subjects, in particular in human subjects.

One aspect of the invention is a method for producing a composition comprising secretory-like immunoglobulin, in particular secretory-like IgA or secretory-like IgM, in vitro, comprising the steps of
(a) Obtaining a blood-derived protein composition comprising J chain-containing IgA or J chain-containing IgM in a non-purified form,
(b) admixing or combining the composition of step (a) with secretory component.

Preferably, the composition of step (a) contains at least 5% (w/w) J chain-containing IgA, more preferably at least 10%, more preferably at least 20%, even more preferably at least 30%, more preferably at least 50%, most preferably it will contain at least 70% J chain-containing IgA. Preferably, the composition of step (a) is derived from human blood, e.g. human plasma or fractions thereof enriched for IgA or even J chain-containing IgA, but no specific purification steps for J chain-containing IgA dimers or polymers is required. Therefore, the J chain-containing IgA will be in a composition also comprising other proteins such as monomeric IgA, IgM, or IgG. For example, the composition may comprise more than 10% monomeric IgA, and/or more than 10% IgG, and/or more than 10% IgM. While an enrichment for J chain-containing dimeric IgA may happen as part of the processing of human plasma fractions for the purification of plasma proteins such as IgG, albumin, alpha-1 antitrypsin, and coagulation factors, no purification step specifically designed to separate J chain-containing dimeric IgA or polymers from other proteins, like affinity chromatography or size exclusion and selection of the fraction of the relevant molecular mass, is necessary to obtain the composition of step (a).

In another preferred aspect of the invention, the composition of step (a) contains at least 5% (w/w) J chain-containing IgM, more preferably at least 10%, more preferably at least 20%, even more preferably at least 30%, more preferably at least 50%, most preferably it will contain at least 70% J chain-containing IgM. Preferably, the composition of step (a) is derived from human blood, e.g. human plasma or fractions thereof enriched for IgM or even J chain-containing IgM, but no specific purification steps for J chain-containing IgM is required. Therefore, the J chain-containing IgM will be in a composition also comprising other proteins such as IgA or IgG. For example, the composition may comprise more than 10% IgA, and/or more than 10% IgG. While an enrichment for J chain-containing IgM may happen as part of the processing of human plasma fractions for the purification of plasma proteins such as IgG, albumin, alpha-1 antitrypsin, and coagulation factors, no purification step specifically designed to separate J chain-containing IgM from other proteins, like affinity chromatography or size exclusion and selection of the fraction of the relevant molecular mass, is necessary to obtain the composition of step (a).

The secretory component comprised in the composition may be recombinant secretory component, preferably secretory component produced in a mammalian cell line. Secretory component is the extracellular portion of the polymeric immunoglobulin receptor (plgR), which usually gets associated during secretion with dimeric or polymeric IgA or pentameric IgM comprising a J chain. J chain-containing IgA/IgM binds to the polymeric immunoglobulin receptor at the basolateral surface of epithelial cells and is taken up into the cell by transcytosis. This receptor complex then transits through the cellular compartments before being secreted on the luminal surface of the epithelial cells. The transcytosed IgA/IgM-plgR complex is then released through proteolysis, and part of the polymeric immunoglobulin receptor (plgR), referred to as the secretory component, stays associated with the J chain-containing IgA/IgM, releasing secretory IgA/IgM. However, there is evidence that reverse transcytosis of IgA, i.e. from the luminal surface to the basolateral surface, can also take place.

The human plgR is cloned and sequenced, its sequence is available as SwissProt entry P01833, and shown in Seq ID NO: 1. Human plgR is a glycoprotein with 764 amino acid residues, containing a signal peptide (residues 1 to 18), an extracellular part (residues 19 to 638), a transmembrane region (residues 639 to 661), and a cytoplasmic region (residues 662 to 764). Residues 19 to 603 are thought to associate with J chain-containing IgA as described above, and this part of this glycoprotein is usually referred to as the secretory component.

The secretory component used in the composition of the invention can comprise any extracellular plgR sequence that is capable of associating with J chain-containing IgA. For example, secretory component may comprise extracellular domains of plgR from mammalian sources, e.g. from primates, cattle, horses, cats, dogs, rabbits, guinea pigs, rats or mice, or variants thereof. Functional hybrids of the extracellular domains from several mammalian species or variants thereof are also contemplated for use in the invention, e.g. prepared by fusing the immunoglobulin-like domains from different species into a secretory component-like protein. A functional secretory component may also be formed by fusing a selection of immunoglobulin-like domains normally present, e.g. rabbit secretory component is functional being composed of only domains 1, 4 and 5. Preferably, however, the human secretory component or functional variants thereof is used.

Therefore the secretory component used in the composition of the invention preferably comprises residues 19 to 603 of SEQ ID NO: 1 or functional variants thereof. Functional variants may include deletions, insertions, and/or substitutions, preferably substitutions are conservative substitutions, e.g. a basic amino acid residue is substituted for another basic amino acid, a hydrophobic amino acid is substituted for another hydrophobic amino acid, etc. The variant secretory component is at least 50% identical in sequence to residues 19 to 603 of SEQ ID NO: 1, preferably at least 55%, 60%, 65%, 70%, 75%, 80%, more preferably at least 85% or even 90%, even more preferably at least 92%, 94%, 95%, 97%, 98%, or even 99% identical to residues 19 to 603 of SEQ ID NO: 1. Preferably, the secretory component comprises the extracellular portion of the plgR, more preferably the extracellular portion of the human plgR, most preferably the secretory component comprises or even consists of residues 19 to 603 of SEQ ID NO: 1.

The skilled person is well aware how to produce the secretory component by recombinant techniques. An example of expression of human secretory component in CHO cells has been described by Phalipon et al (Phalipon A et al (2002) Immunity 17:107-115), but the invention is not limited to secretory component produced by this system. For example, the desired cDNA sequence can be produced synthetically or cloned via RT-PCR, using RNA isolated from cells or tissue expressing plgR as template. The cDNA can then be inserted into a mammalian expression vector such as pcDNA3 - many alternative expression vectors are available. The recombinant expression vector will then be introduced into a suitable host cell line, such as CHO, Cos, HEK293, or BHK. Other cell lines are available and can also be used. Methods for introducing such vectors into a cell line include lipofection, electroporation and other techniques well known to the skilled person. Usually cells harboring the expression vector and expressing the protein of interest are then selected and cloned. Viral expression systems can also be used, for example, vaccinia virus can be used to express proteins at high levels in mammalian cells, baculovirus expression systems can be used to express proteins at high levels in insect cells. Yeast or bacterial expression systems can also be envisaged, and such expression systems are known to the skilled person.

The secretory component or variant thereof used in the composition of the invention may also comprise a tag, such as a hexa-Histidine tag, which can aid in the purification of the resulting protein. If such a tag is attached via a cleavable linker, the tag may be cleaved off prior to use in the invention. Similarly, the secretory component may be produced as a fusion protein. Again, a cleavable linker may be used so that the fusion partner may be cleaved off the secretory component prior to use in the invention.

The skilled person can then purify the expressed protein with standard methods. Recombinant secretory component will be purified to high purity with a suitable method, for example size-exclusion and/or ion exchange chromatography. The final preparation of recombinant secretory component will be essentially free of contaminants, particularly host cell proteins.

The secretory component may also be obtained from a natural source, preferably from milk, saliva or mucus. Preferably the secretory component is of human origin, but secretory component from other species can also be used in the invention.

The molar ratio between secretory component and J chain within IgA dimers/polymers or IgM pentamers in the composition of step (a) ranges between 1:10 and 10:1, preferably between 1:5 and 5:1, more preferably between 1:2 and 2:1.

The molar ratio between secretory component and J chain within the composition of step (a) is between 1:10 and 10:1, preferably between 1:5 and 5:1, more preferably between 1:2 and 2:1.

The amount of secretory component used in step (b) may be at least 1 part (by weight) of secretory component to 50 parts (by weight) of protein in the composition of step (a), preferably at least 1 part to 40, 30, 20, 15, 10, most preferably at least 1 part of secretory component to 5 parts of protein in the composition of step (a).

Another aspect of the invention is a composition comprising secretory-like IgA obtainable by a method of the invention. Yet another aspect of the invention is a composition comprising secretory-like IgM obtainable by a method of the invention. Yet a further aspect is a composition comprising secretory-like IgA and secretory-like IgM obtainable by a method of the invention, for example in a molar ratio of between 10:1 and 1:10, preferably between 5:1 and 1:5, more preferably between 2:1 and 1:2. In another aspect of the invention the combined content of IgA and IgM in the composition exceeds 50%, preferably 60%, more preferably 70%, even more preferably 80%, even more preferably 90%, most preferably it is 100%.

A further aspect of the invention is the composition described above for medical use. For example, the compositions of the invention can be used advantageously to treat necrotizing enterocolitis, and generally infections at mucosal surfaces.

The composition may further comprise one or more pharmaceutically acceptable carrier or excipient, and/or a stabilizer. The composition may be formulated in liquid form, as a syrup, a lotion, an ointment, a powder which may be reconstituted with a liquid prior to administration, a capsule, a pill, a gel, a cream, a jelly, a controlled release formulation, or any other formulation suitable for the intended medical use. For example, for the treatment of GI diseases, the composition may be formulated with a protective coating that dissolves in the desired area of the GI tract to release the composition. The composition may be taken orally, administered topically, enterally, by inhalation, or any other suitable route for the intended use. For oral application acid pump inhibitors may be co-administered.

The proteins in the composition may be concentrated, e.g. using dia/ultrafiltration or other standard methods, prior to being formulated. In addition, the composition may be lyophilized, and then reconstituted with a suitable solution prior to use.

### Definitions

The term "secretory-like IgA" or "secretory-like plasma IgA" is intended to encompass J chain-containing (plasma) IgA combined with a protein that is secretory component or a functional variant thereof, which serves to provide some protection from proteolytic digestion. Typically, the J chain-containing IgA will comprise two or four, or even more IgA monomers. Typically, the J chain-containing IgA will be mixed with a secretory component or variant thereof in vitro, i.e. the association between secretory component and J chain-containing IgA takes place in vitro rather than during transcytosis.

The term "secretory-like IgM" is intended to encompass J chain-containing (plasma) IgM combined with secretory component or a functional variant thereof in vitro. Preferably, the J chain-containing IgM will be pentameric IgM.

"Specific purification steps for J chain-containing IgA dimers or polymers" relate to purification steps that would be included in a purification process specifically designed to separate J chain-containing IgA dimers or polymers from other proteins, such as monomeric IgA, other immunoglobulins, and other plasma proteins. Such specific purification steps may, for example, include affinity chromatography with a ligand specifically binding to J chain, or size exclusion chromatography selecting the fractions containing proteins of a molecular weight corresponding to J chain-containing IgA dimers. While the process for preparing the composition from plasma may comprise methods that lead to an enrichment of IgA or even J chain-containing IgA, such as ion exchange chromatography, the J chain-containing IgA is not specifically purified. Thus "J chain-containing IgA in a non-purified form" refers to a composition containing less than 80% J chain-containing IgA, typically less than 70%, 60%, or 50% J chain-containing IgA, it may even contain less than 40%, 30%, 25%, 20%, 15% or even 10% J chain containing IgA.

"Specific purification steps for J chain-containing IgM" relate to purification steps that would be included in a purification process specifically designed to separate J chain-containing IgM from other proteins, such as other immunoglobulins, and other plasma proteins. Such specific purification steps may, for example, include affinity chromatography with a ligand specifically binding to IgM or to J chain, or size exclusion chromatography selecting the fractions containing proteins of a molecular weight corresponding to J chain-containing IgM pentamers. While the process for preparing the composition from plasma may comprise methods that lead to an enrichment of J chain-containing IgM, such as ion exchange chromatography, the J chain-containing IgM is not specifically purified. Thus "J chain-containing IgM in a non-purified form" refers to a composition containing less than 80% J chain-containing IgM, typically less than 70%, 60%, or 50% J chain-containing IgM, it may even contain less than 40%, 30%, 25%, 20%, 15% or even 10% J chain containing IgM.

"Secretory component" or variants thereof relates to the extracellular portion of the plgR, or functional variants thereof. As detailed above, the most preferred secretory component is human secretory component, e.g. corresponding to residues 19 to 603 of SEQ ID NO: 1. However, amino acid deletions, insertions, substitutions may be included, as long as they lead to a functional protein, i.e. one that is still capable of associating with J chain-containing IgA. Homologues from other mammalian species are also included, as are chimeric proteins comprising parts from different species.

The term "% [percent]" when used to describe the content of immunoglobulin in a composition/preparation means weight per weight protein.

### List of Figures

The invention will now be illustrated by the following, non-limiting examples, with reference to the following figures and sequence listing:
Figure 1 shows a diagram of the structure of monomeric, dimeric J chain-containing and secretory IgA.
Figure 2 shows Western blots of different IgA preparations, developed with different antibodies:
   - Fig 2A shows a Western blot of different IgA preparations, developed with anti-α chain, anti-J chain or anti-secretory component antibody.
   - Fig 2B shows a Western blot of different secretory-like and secretory IgA preparations, developed with anti-secretory component antibody.
   - Fig 2C shows a Western blot of different size exclusion chromatography fractions, developed with antibodies to secretory component, α chain and J chain.
   - Fig 2D shows a chromatogram of a size exclusion chromatography run of IgAF5.
Figure 3 shows dot blots, using immobilized secretory component:
   - Fig 3A shows a flow diagram of how the assay was set up
   - Fig 3B shows SC capturing J chain-containing IgA
   - Fig 3C shows SC capturing J chain-containing IgM
Figure 4 shows Western blots of time course experiments of different IgA preparations (A) or IgM preparations (B) incubated with intestinal washes. The blots were developed using anti-heavy chain antibodies.

SEQ ID NO: 1 shows the protein sequence of human plgR.

### Examples

### Example 1: Western blot of IgA preparations from plasma mixed with recombinant secretory component

### Materials and Methods

### 1.1 IgA preparation from plasma by affinity chromatography and/or by sequential elution of MPHQ column

Human plasma IgA was purified by affinity chromatography using CaptureSelect Human IgA resin (Bioaffinity Company BAC, Naarden, Netherlands) according to the resin manufacturer's protocol using 3 different sources of plasma IgA as starting material, namely cryo-depleted plasma, re-solubilised cold ethanol fractionation paste, or a strip fraction from an anion-exchange (AIEX) chromatography column obtained by sanitizing said column, according to the commercially applied IgG purification process of CSL Behring AG (Berne, Switzerland). Briefly, cryo-depleted pool plasma, re-solubilised paste or AIEX strip fraction was diluted in phosphate buffered saline (PBS) to an IgA concentration of approximately 1 mg/mL and then loaded onto a PBS-equilibrated CaptureSelect Human IgA column, without exceeding the IgA binding capacity of the column. After loading the column was washed with PBS, and IgA was eluted with glycin buffer at pH 3. The eluate was adjusted with 0.5M Tris (pH 8) to pH 4.5 and concentrated up to 16 mG/mL protein in PBS. SlgA from human milk was purified by the same method.

From the AIEX chromatography step of the IVIg manufacture process of CSL Behring AG (Berne, Switzerland), fraction F4 was obtained after a post-wash of the Macro-Prep High Q (Bio-Rad, Hercule, CA) column with 10 mM phosphate / 30 mM acetate at pH 6.5 by elution with 55 mM tartrate / 5 mM acetate at pH 7.6. Fraction F5 was subsequently eluted with 50 mM phosphate / 25 mM citrate at pH 5.0. F4 and F5 were brought to approximately 1 mG/mL in PBS by ultra-/diafiltration, and then depleted of IgG by affinity chromatography using IgSelect resin (GE Healthcare, Glattbrugg, Switzerland). IgA F4 was directly harvested in the flowthrough of the IgSelect chromatography of F4 load. To obtain IgA F5, the IgSelect flowthrough of F5 load was depleted of IgM by affinity chromatography using CaptureSelect Human IgM resin (Bioaffinity Company BAC). IgA F4 and IgA F5 were brought to final concentrations by ultra-/diafiltration.

### 1.2 IgM preparation from plasma by affinity chromatography

Human plasma IgM was purified by affinity chromatography using CaptureSelect Human IgM resin (Bioaffinity Company BAC, Naarden, Netherlands) according to the resin manufacturer's protocol using the same 3 different sources as starting material as described in section 1.1 for IgA, namely cryo-depleted plasma, re-solubilised cold ethanol fractionation paste, or a strip fraction from an anion-exchange (AIEX) chromatography column obtained by sanitizing said column, according to the commercially applied IgG purification process of CSL Behring AG (Berne, Switzerland). Briefly, cryo-depleted pool plasma, re-solubilised paste or AIEX strip fraction was diluted in phosphate buffered saline (PBS) to an IgM concentration of approximately 1 mg/mL and then loaded onto a PBS-equilibrated CaptureSelect Human IgM column, without exceeding the IgM binding capacity of the column. After loading the column was washed with PBS, and IgM was eluted with glycin buffer at pH 3. The eluate was adjusted with 0.5M Tris (pH 8) to pH 4.5 and concentrated up to 10 mG/mL protein in PBS.

### 1.3. Western blots

SDS-PAGE and electrotransfer onto nitrocellulose (NC) membranes were carried out using the Mini-Cell system from Invitrogen (Carlsbad, CA), according to the manufacturer's protocols. Briefly, samples were denatured in sample buffer under reducing or non-reducing conditions, respectively, and electrophoretically separated on pre-cast gradient gels, NuPAGE Novex Bis-Tris 4-12% 1.0 mm 10 well, using NuPAGE MOPS Electrophoresis Buffer (Invitrogen). Wet transfer onto NC membranes (0.2 µm) was performed with the XCell II Blot Module (Invitrogen) and NuPAGE Transfer Buffer. The membranes were then blocked for 30 min in PBS-0.5% Tween 20 solution (PBS-T) containing 4% Rapilait skim milk powder (Migros, Switzerland). For immunoblotting polyclonal rabbit antibodies were used: 1) rabbit anti-human alpha chain (Dako, horseradish peroxidase (HRP)-conjugated: 1/5'000 dilution); 2) rabbit anti-human J chain (BioGenex, Fremont, CA; 1/300 dilution), followed by secondary anti-rabbit HRP-conjugated antiserum (Sigma; 1/10'000 dilution); 3) rabbit anti-human SC (Dako; 1/5000 dilution), followed by secondary anti-rabbit HRP-conjugated antiserum (Sigma; 1/10'000 dilution). All incubations were performed in PBS-T containing 4% milk powder at ambient temperature for 1-2 hours. After final washing with PBS-T, immunodetection on membranes was revealed by chemiluminescence and digitally recorded in an ImageQuant LAS 4000 system (GE Healthcare Lifesciences).

### 1.4 Association of plasma derived IgA with recombinant secretory component

Secretory-like IgA was obtained by combining in vitro 100 mg of IgA F5 with 4 mg of recombinant human secretory component (recSC). Association was performed in PBS for 30 min at room temperature as previously described in (Crottet, P., and Corthésy, B. (1998) J. Immunol. 161:5445-5453).

### 1.5 Size exclusion chromatography (SEC) fractionation

IgA F5 comprising secretory-like IgA (associated *in vitro* with recSC) was injected at 200 µG/ 20µL into an Agilent Technologies 1050 HPLC system for size exclusion chromatography at a flowrate of 1.5 mL/min over a TSKgel G3000SWXL 7.8 mm ID x 30 cm column (Tosoh Bioscience). Fractions of 0.75 mL were collected between 8.0 and 13.5 min retention time in intervals of 30 sec.

### Results

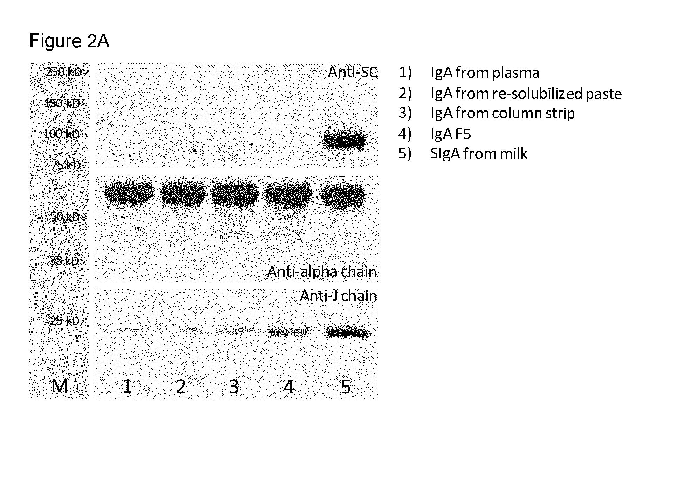

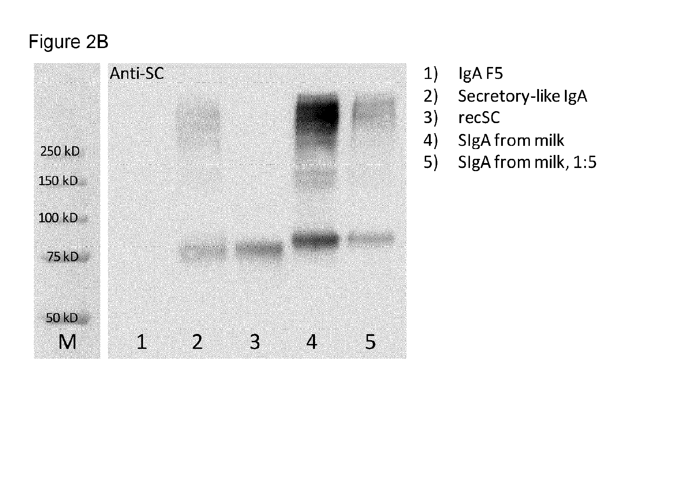

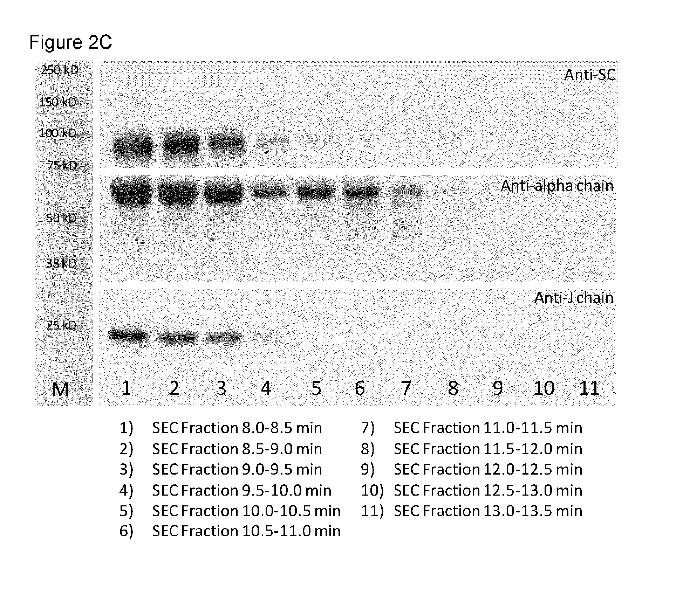

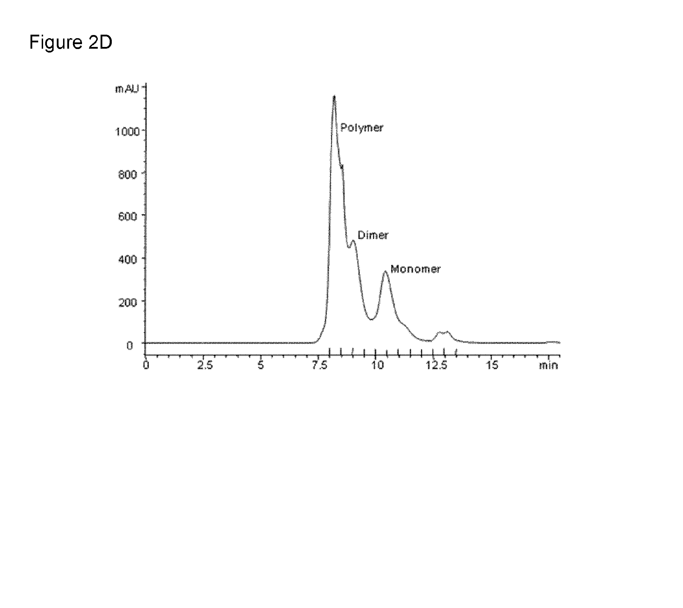

The results are shown in Figure 2. Figure 2A demonstrates a comparison of IgA purified by affinity chromatography from plasma, from re-solubilised paste and from AIEX strip fraction or by sequential elution to obtain IgAF5 as described in 1.1 with SlgA from human milk. Secretory component was found in SlgA from milk but not in any of the IgA fractions purified from human plasma. All preparations contained the same amount of IgA heavy chain/alpha-chain. As expected, SlgA from milk contained the highest amount of J chain as essentially all IgA molecules are expected to be present as J chain-containing dimers. The amount of J chain and thus J chain-containing IgA dimers in IgA purified from plasma was low. This is expected as only a small portion of plasma IgA is present in dimeric form. A similar content of J chain was observed in IgA purified from re-solubilised paste. Surprisingly an increased fraction of IgA was present as J chain-containing dimers in the column strip fraction as evidenced by the increased amount of J chain. Surprisingly, this was further increased in IgA F5. This accumulation occurred without application of a specific process step for enrichment.

Figure 2B shows that no secretory-component containing IgA was present in IgA F5. After association with recSC free recSC (75kDa) and dimeric IgA associated with recSC were found. Indeed the secretory-like plasma IgA appeared similar to SlgA from milk. It was estimated that in the preparation of IgAF5 used in the shown experiment the content of J chain-containing IgAF5 was about 20%. Indeed, the signal strength observed in lane 2 was comparable to the signal of 1 :5-diluted human milk SlgA.

Figure 2C shows the content of SC, IgA alpha chain and J chain in fractions obtained by size-exclusion chromatography of secretory-like IgAF5. recSC was observed in early fractions corresponding to high molecular weight forms of IgA - likely polymeric and dimeric forms. Appearance of SC coincided with appearance of J chain, indicating that indeed the SC-containing fraction of IgAF5 was the dimeric, J chain-containing fraction. In addition IgA alpha-chain was detected in fractions of smaller molecular weight, likely comprising the monomeric fraction of IgAFS; these fractions were devoid of SC and J chain. These data demonstrate that recSC admixed with plasma-derived IgA containing monomeric and dimeric forms of IgA specifically associated with the dimeric, J chain-containing forms of IgA.

Figure 2D shows the chromatogram of the SEC run during which fractions were collected between retention time 8.0 min and 13.5 min. Peaks representing IgA polymers, dimers and monomers are indicated.

### Example 2: Dot blot re-association assay (DORA)

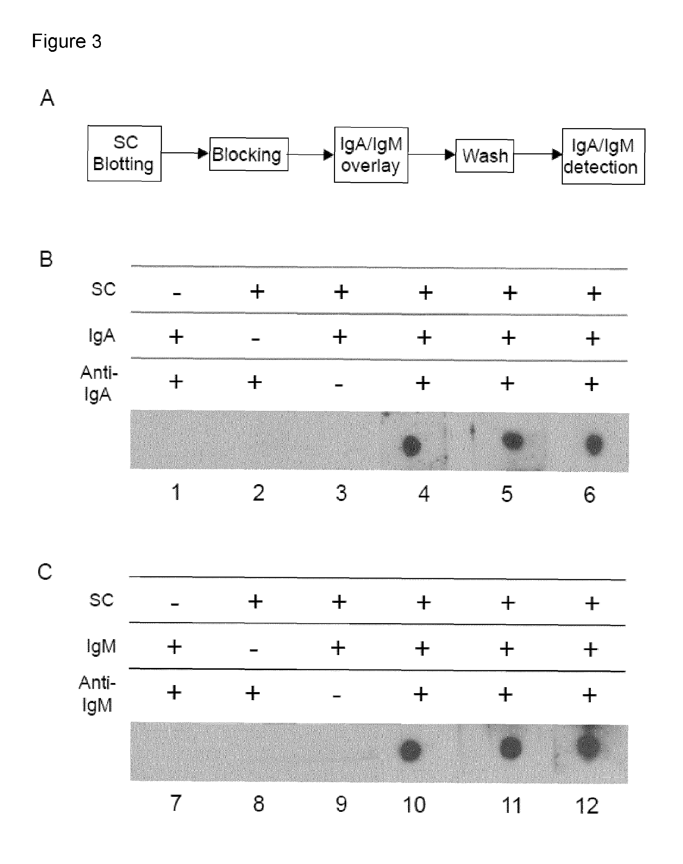

A dot-blot re-association assay was used to show the association of immobilised secretory component with plasma-derived IgA or IgM *in vitro.* Briefly, as shown in Figure 3A, secretory component was dotted onto blotting membranes; non-specific binding sites were blocked. Thereafter plasma-derived IgA (Figure 3B) or IgM (Figure 3C) obtained by affinity chromatography from plasma (lane 4), from re-solubilised paste (lane 5) or from AIEX strip fraction (lane 6) obtained as described in 1.1 and 1.2 were applied to the membrane. After washing off unbound IgA or IgM, bound IgA/IgM was detected as described briefly below.

DORA was carried out essentially as described (Rindisbacher, L. et al (1995) J. Biol. Chem. 270:14220-14228), with the following modifications: Blotting membranes consisted of polyvinylidone fluoride (PVDF) polymer, blocking solution was phosphate-buffered saline-0.05% Tween-20 (PBS-T) containing 1% bovine serum albumin (BSA), crude preparations enriched in IgA were used for overlay incubation in 200 µl of PBS-T containing 0.1 % BSA, and detection antibodies were directly coupled to HRP.

The results for IgA are shown in Figure 3B. Immobilised secretory component was capable of capturing plasma-derived IgA. Similar to what is shown in Figure 2C this demonstrates that recSC associated with plasma-derived IgA dimers.

The results for IgM are shown in Figure 3C. Immobilised secretory component was capable of capturing plasma-derived IgM. This demonstrates that recSC associated with plasma-derived IgM.

### Example 3: Digestion of secretory-like IgA and secretory-like IgM with intestinal washes

In order to prove a functional advantage of association of purified secretory component with J chain-containing IgA and IgM, respectively, IgA and IgM were prepared as described in paragraph 1.1 and 1.2. Secretory-like IgA was obtained by enriching J chain-containing IgA using size-exclusion chromatography and combining *in vitro* 10 µg thereof with 2.5 µg of recombinant human secretory component (hSCrec). Secretory-like IgM was obtained by enriching pentameric IgM using size-exclusion chromatography and combining *in vitro* 25 µg thereof with 2.5 µg of recombinant human secretory component (hSCrec). Association was performed in PBS for 30 min at room temperature as previously described (Crottet, P., and Corthésy, B. (1998) J. Immunol. 161:5445-5453). Integrity and proper assembly of the molecules into possibly covalent complexes were examined by SDS-PAGE under non-reducing and reducing conditions, followed by Western blotting and immunodetection with antiserum specific for hSC as indicated above.

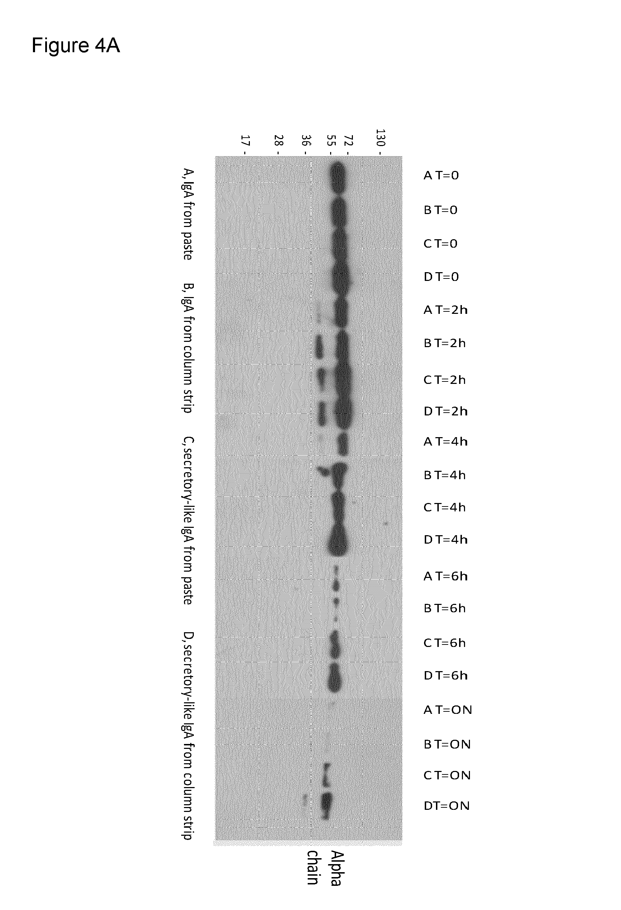

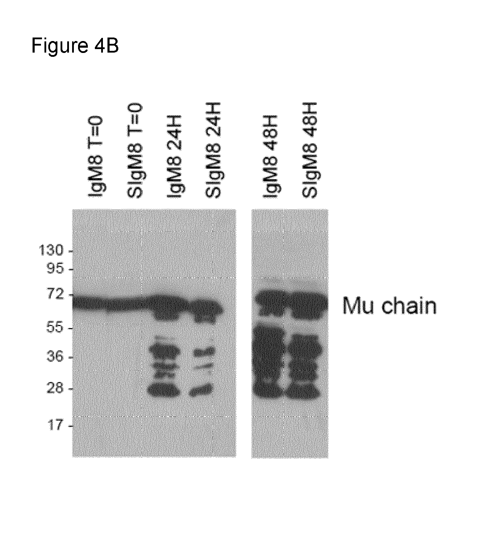

Collection of intestinal washes from BALB/c mice (4-6 weeks old) was done according to the published procedure (Crottet, P., and Corthésy, B. (1998) J. Immunol. 161:5445-5453). For in vitro digestion, 120 ng of purified J chain-containing IgA and reconstituted secretory-like IgA were mixed (or not) with 1 or 2 µl of intestinal washes in a final volume of 20 µl of PBS and incubated at 37°C for various periods of time as indicated in Figure 4 (T= time in hours). Reactions were stopped by the addition of 2 µl of Complete^{™} protease inhibitor mixture (Roche Applied Science, Rotkreuz, Switzerland), and kept frozen until analysis by Western blot detecting the reduced form of heavy chain of the antibody.

The results are shown in Figure 4A and B. IgA from re-solubilized paste and from column strip obtained as described in 1.1. and IgM from column strip as described in 1.2. were either used as such or both after association with recSC to form secretory-like IgA (Fig. 4A) or secretory-like IgM (Fig. 4B). For IgA, after 4 hours of digestion with intestinal enzymes the signal of non-associated IgA started to decrease, indicative of proteolytic digestion; the effect was stronger after 6 hours and after overnight digestion, no intact IgA alpha-chain was detected by Western blot. In contrast, secretory-like IgA was much less sensitive to digestion by intestinal proteases and even after overnight exposure a significant portion of IgA alpha-chain within secretory-like IgA remained intact.

For IgM (Fig. 4B) a comparison of IgM and freshly associated secretory-like IgM (SlgM) with the same preparations after 24h and 48h of digestion is shown. Appearance of degraded mu-chain fragments occurred more rapidly and more extensively for IgM compared to SlgM, confirming for IgM - similar as for IgA - that association with SC provided improved structural stability.

Overall, this demonstrates that the specific association with recSC provided improved structural stability, and will make the digestion-prone plasma IgA molecules fit for mucosal application, e.g. via the oral route.

### Example 4: Shigella

The human colonic adenocarcinoma epithelial Caco-2 cell line (American Type Tissue Collection) is seeded on polyester Snapwell filters (diameter, 12 mm; pore size, 0.4 µm; Corning Costar) as described (Crottet, S., Corthésy-Theulaz, I., Spertini, F., and Corthésy, B. (2002) J. Biol. Chem. 277:33978-33986). The integrity of the polarized Caco-2 cell monolayer is checked by measuring the transepithelial electrical resistance (TER) using a Millicell-ERS device (Millipore). TER values of well-differentiated monolayers range between 450-550 Ω x cm².

2 x 10⁷ bacteria are mixed with 100 µg of IgA or 125 µg of secretory-like IgA or 250 µg of IgM or secretory-like IgM in a final volume of 500 µl of plain DMEM (P-DMEM: DMEM complemented with 10 mM HEPES, 20 µg/ml transferrin, 2 mM glutamine, 1% non-essential amino acids, 1 mM sodium pyruvate) and incubated for 1 h at RT under gentle agitation. The mixtures are resuspended in P-DMEM to infect polarized Caco-2 cell monolayers.
1 h before the use of polarized Caco-2 cell monolayers, C-DMEM is replaced by P-DMEM in both the apical and basolateral compartments. Apical medium is then replaced by 500 µl of bacterial suspensions (2 x 10⁷ bacteria) as such or in combination with the antibody. TER values are measured at selected time-points from the beginning of the infection onward.

To examine the integrity of Caco-2 cell monolayers, Snapwells are washed with PBS, prior to fixation overnight with 5 ml of 4% paraformaldehyde at 4°C. After washing with PBS, filters are permeabilized and non-specific binding sites are blocked using PBS containing 5% FCS and 0.2% Triton X-100 (PBS-Tr) for 30 min at RT. All Ab are diluted in PBS-Tr. Filters are incubated with rabbit anti-human ZO-1 (1/200, Invitrogen) for 2 h at RT, washed in PBS, followed by goat anti-rabbit IgG conjugated with Alexa Fluor® 647 (1/100, Invitrogen) for 90 min at RT. To visualize cells, filters are finally incubated with 100 ng/ml of 4',6-diamidino-2-phenylindole (DAPI) in PBS (Invitrogen) for 30 min. Filters are cut out of their holders, and mounted in Vectashield solution for observation using a Zeiss LSM 710 Meta confocal microscope (Carl Zeiss, Germany) equipped with either a 10x or a 40x objective. Images are processed using the Zeiss ZEN 2009 light software.

### Result

Exposure of the intestinal epithelial cell monolayer to Shigella leads to disruption of the integrity of the monolayer, evidenced by a decrease of TER and by a visibly compromised cell monolayer as observed by confocal microscopy. Addition of secretory-like IgA/IgM delays and partially inhibits the destruction of the monolayer, indicated by a significant inhibition of the reduction of TER and by a more intact cell monolayer by confocal microscopy. Plasma IgA/IgM alone is not effective (or at least not as effective) as secretory-like IgA/IgM in the prevention of Shigella infection.

### Example 5: Prevention of recurrence of Clostridium difficile infection (CDI)

The composition of the invention is used in a mouse model of *Clostridium difficile* infection.

C57BL/6 mice are treated with a mixture of oral antibiotics (kanamycin, gentamicin, colistin, metronidazole, and vancomycin) for 3 days as previously described (Chen X, et al. Gastroenterology 2008 Dec;135(6):1984-92). Two days later, they are given parenteral clindamycin phosphate (10 mg/kg s.c.) [Day -1]. One day later [Day 0] they are challenged by gavage with 0.5 x 10⁵ cfu of toxinogenic *C. difficile* strain 10465. A moderate to fulminant colitis develops 1 to 5 days after the administration of *C. difficile.* Untreated, this progresses rapidly into severe and fatal colitis in the majority of animals. To treat primary infection animals receive vancomycin, for 5 days after *C. difficile* challenge, and the animals are monitored for mortality, as well as the presence or absence of severe CDI with diarrhea. Animals judged to be in a moribund state are euthanized by a single injection of sodium pentobarbital. To study recurrence of CDI animals surviving primary *C. difficile* challenge are maintained under observation until day 28. Animals are weighed 3 times weekly from day 7 to 28. After cessation of vancomycin treatment animals receive IgA or secretory-like IgA (400 mg/kg body weight via the oral route) for 5 days starting the day after the last dose of vancomycin.

### Result

Animals treated with vancomycin survive the primary infection with *C. difficile.* However, a significant proportion of animals - up to 70% - succumb to recurrence of *C. difficile* infection within 3-4 days after termination of vancomycin treatment. In contrast, recurrence of infection is prevented if animals are treated with secretory-like IgA via the oral route. Plasma IgA alone is not effective (or at least not as effective) as secretory-like IgA in preventing recurrence of *C. difficile* infection.

## Claims

1. Method for producing a composition comprising secretory-like immunoglobulin in vitro, comprising the steps of
(a) Obtaining a blood-derived protein composition comprising J chain-containing immunoglobulin in a non-purified form,
(b) admixing the composition of step (a) with secretory component.

2. The method of claim 1, wherein the secretory-like immunoglobulin is secretory-like IgA and/or secretory-like IgM.

3. The method of claim 1 or claim 2, wherein the composition of step (a) contains at least 5% J chain-linked IgA.

4. The method of claim 3, wherein the composition of step (a) contains at least 10% J chain-linked IgA.

5. The method of claim 3, wherein the composition of step (a) contains at least 20% J chain-linked IgA.

6. The method of claim 3, wherein the composition of step (a) contains at least 30% J chain-linked IgA.

7. The method of claim 3, wherein the composition of step (a) contains at least 50% J chain-linked IgA.

8. The method of any previous claim, wherein the composition of step (a) is derived from human blood.

9. The method of any previous claim, wherein the secretory component in step (b) is recombinant secretory component.

10. The method of any previous claim, wherein the secretory component is human secretory component.

11. The method of any previous claim, wherein the secretory component is produced in a mammalian cell line.

12. The method of any previous claim, wherein the secretory component is the extracellular portion of the polymeric immunoglobulin receptor plgR.

13. The method of any previous claim, wherein in step (b) the molar ratio between added secretory component and J chain within IgA dimers/polymers ranges between 1:10 and 10:1.

14. The method of claim 13, wherein the ratio ranges between about 1:5 and 5:1.

15. The method of claim 14, wherein the ratio ranges between about 1:2 and 2:1.

16. A composition comprising secretory-like IgA and/or secretory-like IgM or a combination thereof obtainable by a method of any of claims 1 to 15.

17. The composition of claim 16, further comprising a pharmaceutically acceptable carrier or excipient.

18. The composition of claim 16 or claim 17 for medical use.


SEQUENCE LISTING

<110>  CSL Behring AG

<120>  Compositions

<130>  A192

<160>  1

<170>  PatentIn version 3.3

<210>  1
<211>  764
<212>  PRT
<213>  Homo sapiens

<400>  1

Met Leu Leu Phe Val Leu Thr Cys Leu Leu Ala Val Phe Pro Ala Ile
1               5                   10                  15

Ser Thr Lys Ser Pro Ile Phe Gly Pro Glu Glu Val Asn Ser Val Glu
            20                  25                  30

Gly Asn Ser Val Ser Ile Thr Cys Tyr Tyr Pro Pro Thr Ser Val Asn
        35                  40                  45

Arg His Thr Arg Lys Tyr Trp Cys Arg Gln Gly Ala Arg Gly Gly Cys
    50                  55                  60

Ile Thr Leu Ile Ser Ser Glu Gly Tyr Val Ser Ser Lys Tyr Ala Gly
65                  70                  75                  80

Arg Ala Asn Leu Thr Asn Phe Pro Glu Asn Gly Thr Phe Val Val Asn
                85                  90                  95

Ile Ala Gln Leu Ser Gln Asp Asp Ser Gly Arg Tyr Lys Cys Gly Leu
            100                 105                 110

Gly Ile Asn Ser Arg Gly Leu Ser Phe Asp Val Ser Leu Glu Val Ser
        115                 120                 125

Gln Gly Pro Gly Leu Leu Asn Asp Thr Lys Val Tyr Thr Val Asp Leu
    130                 135                 140

Gly Arg Thr Val Thr Ile Asn Cys Pro Phe Lys Thr Glu Asn Ala Gln
145                 150                 155                 160

Lys Arg Lys Ser Leu Tyr Lys Gln Ile Gly Leu Tyr Pro Val Leu Val
                165                 170                 175

Ile Asp Ser Ser Gly Tyr Val Asn Pro Asn Tyr Thr Gly Arg Ile Arg
            180                 185                 190

Leu Asp Ile Gln Gly Thr Gly Gln Leu Leu Phe Ser Val Val Ile Asn
        195                 200                 205

Gln Leu Arg Leu Ser Asp Ala Gly Gln Tyr Leu Cys Gln Ala Gly Asp
    210                 215                 220

Asp Ser Asn Ser Asn Lys Lys Asn Ala Asp Leu Gln Val Leu Lys Pro
225                 230                 235                 240

Glu Pro Glu Leu Val Tyr Glu Asp Leu Arg Gly Ser Val Thr Phe His
                245                 250                 255

Cys Ala Leu Gly Pro Glu Val Ala Asn Val Ala Lys Phe Leu Cys Arg
            260                 265                 270

Gln Ser Ser Gly Glu Asn Cys Asp Val Val Val Asn Thr Leu Gly Lys
        275                 280                 285

Arg Ala Pro Ala Phe Glu Gly Arg Ile Leu Leu Asn Pro Gln Asp Lys
    290                 295                 300

Asp Gly Ser Phe Ser Val Val Ile Thr Gly Leu Arg Lys Glu Asp Ala
305                 310                 315                 320

Gly Arg Tyr Leu Cys Gly Ala His Ser Asp Gly Gln Leu Gln Glu Gly
                325                 330                 335

Ser Pro Ile Gln Ala Trp Gln Leu Phe Val Asn Glu Glu Ser Thr Ile
            340                 345                 350

Pro Arg Ser Pro Thr Val Val Lys Gly Val Ala Gly Gly Ser Val Ala
        355                 360                 365

Val Leu Cys Pro Tyr Asn Arg Lys Glu Ser Lys Ser Ile Lys Tyr Trp
    370                 375                 380

Cys Leu Trp Glu Gly Ala Gln Asn Gly Arg Cys Pro Leu Leu Val Asp
385                 390                 395                 400

Ser Glu Gly Trp Val Lys Ala Gln Tyr Glu Gly Arg Leu Ser Leu Leu
                405                 410                 415

Glu Glu Pro Gly Asn Gly Thr Phe Thr Val Ile Leu Asn Gln Leu Thr
            420                 425                 430

Ser Arg Asp Ala Gly Phe Tyr Trp Cys Leu Thr Asn Gly Asp Thr Leu
        435                 440                 445

Trp Arg Thr Thr Val Glu Ile Lys Ile Ile Glu Gly Glu Pro Asn Leu
    450                 455                 460

Lys Val Pro Gly Asn Val Thr Ala Val Leu Gly Glu Thr Leu Lys Val
465                 470                 475                 480

Pro Cys His Phe Pro Cys Lys Phe Ser Ser Tyr Glu Lys Tyr Trp Cys
                485                 490                 495

Lys Trp Asn Asn Thr Gly Cys Gln Ala Leu Pro Ser Gln Asp Glu Gly
            500                 505                 510

Pro Ser Lys Ala Phe Val Asn Cys Asp Glu Asn Ser Arg Leu Val Ser
        515                 520                 525

Leu Thr Leu Asn Leu Val Thr Arg Ala Asp Glu Gly Trp Tyr Trp Cys
    530                 535                 540

Gly Val Lys Gln Gly His Phe Tyr Gly Glu Thr Ala Ala Val Tyr Val
545                 550                 555                 560

Ala Val Glu Glu Arg Lys Ala Ala Gly Ser Arg Asp Val Ser Leu Ala
                565                 570                 575

Lys Ala Asp Ala Ala Pro Asp Glu Lys Val Leu Asp Ser Gly Phe Arg
            580                 585                 590

Glu Ile Glu Asn Lys Ala Ile Gln Asp Pro Arg Leu Phe Ala Glu Glu
        595                 600                 605

Lys Ala Val Ala Asp Thr Arg Asp Gln Ala Asp Gly Ser Arg Ala Ser
    610                 615                 620

Val Asp Ser Gly Ser Ser Glu Glu Gln Gly Gly Ser Ser Arg Ala Leu
625                 630                 635                 640

Val Ser Thr Leu Val Pro Leu Gly Leu Val Leu Ala Val Gly Ala Val
                645                 650                 655

Ala Val Gly Val Ala Arg Ala Arg His Arg Lys Asn Val Asp Arg Val
            660                 665                 670

Ser Ile Arg Ser Tyr Arg Thr Asp Ile Ser Met Ser Asp Phe Glu Asn
        675                 680                 685

Ser Arg Glu Phe Gly Ala Asn Asp Asn Met Gly Ala Ser Ser Ile Thr
    690                 695                 700

Gln Glu Thr Ser Leu Gly Gly Lys Glu Glu Phe Val Ala Thr Thr Glu
705                 710                 715                 720

Ser Thr Thr Glu Thr Lys Glu Pro Lys Lys Ala Lys Arg Ser Ser Lys
                725                 730                 735

Glu Glu Ala Glu Met Ala Tyr Lys Asp Phe Leu Leu Gln Ser Ser Thr
            740                 745                 750

Val Ala Ala Glu Ala Gln Asp Gly Pro Gln Glu Ala
        755                 760